# EUROPEAN PATENT APPLICATION

(11) **EP 1 371 632 A1**
(43) Date of publication of application: **17.12.2003**
(21) Application number: 03253473.7
(22) Date of filing: 03.06.2003
(51) Int. Cl.: C07C 253/00, C07C 255/16

(54) **Process for producing acetone cyanohydrin**

(30) Priority: 14.06.2002 US 389015 P
(71) Applicant: ROHM AND HAAS COMPANY, Philadelphia, Pennsylvania 19106-2399 (US)
(72) Inventor: Decourcy, Michael Stanley, Houston, Texas 77059 (US)
(74) Representative: Kent, Venetia Katherine

(57) **Abstract**

An improved process for the production of acetone cyanohydrin by the reaction of acetone and HCN under basic pH conditions comprises supplying a metal cyanide composition and an HCN composition to the reaction.

## Description

The process for conversion of acetone cyanohydrin - H₂SO₄ to methacrylates, such as methyl methacrylate or methacrylic acid, has been practiced commercially since 1937 and is based on technology patented by ICI in 1934 (British Patent No. 405,699). Although numerous improvements and alternative production methods have been proposed over the years, the acetone cyanohydrin (ACH) route remains the most common production method for methacrylates and nearly all of the ACH produced in the world is consumed in the production of methacrylates. Because of this, the great majority of ACH production facilities are located adjacent to methacrylate production facilities.

All commercial ACH is prepared via the reaction of acetone and hydrogen cyanide. Because of increasing demand for methacrylates, in recent years, as well as ongoing pressure to minimize manufacturing costs, manufacturers would greatly welcome improvements to the ACH production process which improve production capacity, lower costs and provide safer operation.

Commercially, high purity HCN (at least 90% pure) for making ACH can be produced in one of three ways:
1. By the Andrussow process
2. By the Degussa B-M-A process
3. As a by-product of acrylonitrile production

The most common process, known as the Andrussow process, involves the reaction of natural gas (primarily methane) with ammonia, in the presence of oxygen or air, over a precious metal catalyst. The products of this reaction are a diluted stream of hydrogen cyanide, water, hydrogen, carbon dioxide, carbon monoxide, as well as the unreacted excess quantities of ammonia and methane, and, possibly, nitrogen which arises from the use of air as an oxygen source. Other minor contaminants may include nitrites formed in the reaction. In most cases, the gas stream is purified by isolating the hydrogen cyanide to a purity of about 99%.

A second reaction involving natural gas and ammonia was developed by Degussa and is known as the B-M-A process. The natural gas and ammonia are reacted over a precious metal catalyst, but in the absence of oxygen. The products of this reaction are similar to those of the Andrussow process, except that there is no water, carbon dioxide or carbon monoxide. The hydrogen cyanide is normally purified as anhydrous hydrogen cyanide through distillation before it is reacted to form other cyanide derivatives or metal cyanides.

In a third process, hydrogen cyanide is formed as a by-product or co-product in the production of acrylonitrile by the reaction of propylene and ammonia in the presence of air. Approximately one pound of hydrogen cyanide is produced for every ten or eleven pounds of acrylonitrile produced. It is isolated as about 99% anhydrous hydrogen cyanide before being reacted with caustic or other materials.

Because of the highly cost-dependent market for methacrylates, it would be advantageous to utilize multiple sources of HCN to ensure adequate raw material is available for ACH production. In this way, ACH production rates can be maximized and can better keep pace with end-use methacrylates production demand. Although ACH and methacrylates production facilities are generally located adjacent to one another, the use of multiple HCN sources could result in at least some of the required HCN being produced far from the ACH production site; this situation is particularly common when the HCN is derived as a by-product of an acrylonitrile production process. Because of its toxicity, the safe transport of HCN to a distant ACH/methacrylates production site is an area of major concern for chemical manufacturers.

The following methods for transporting HCN have been proposed:
1. Purify the HCN and ship to a distant user
   - transportation via truck, rail or waterway raises significant safety issues.
2. Convert the HCN to sodium cyanide, ship the sodium cyanide to the user, acidify the sodium cyanide after arrival to release HCN (this may include the optional extra step of distilling the released HCN to remove impurities (see Published International Patent Application No. WO 97/45369 A1)
   - this method is energy and capital intensive
3. Convert the HCN to ACH at the source, then ship the ACH to a distant user
   - transportation via truck, rail or waterway raises significant safety issues (somewhat less hazardous than 100% HCN but nonetheless significant transportation safety issues)
   - high capital cost (building another ACH production unit at the HCN source is capital intensive and may result in inefficient use of total ACH production capacity)

It is the intent of the present invention to provide an improved process for the production of ACH. In this regard, the present invention provides, in one aspect, an improved process for the production of acetone cyanohydrin by the reaction of acetone and HCN under basic pH conditions, the improvement comprising: supplying a metal cyanide composition and an HCN composition to the reactor.

In another aspect, the present invention provides a process for the production of crude acetone cyanohydrin, wherein the process comprises: feeding a metal cyanide to a reactor; feeding a hydrogen cyanide composition to the reactor; feeding acetone to the reactor; maintaining a temperature of between 0°C and 50°C in the reactor; maintaining a pH of at least 7.0 in the reactor; maintaining a residence time in the reactor of between 15 minutes and 120 minutes; recovering a product stream from the reactor comprising crude acetone cyanohydrin.

The production of acetone cyanohydrin suitable for use in the production of methacrylates involves three primary steps: a reaction to form crude ACH; stabilization of the crude ACH; and purification of the crude ACH to product ACH.

In the reaction step, according to the present invention, crude ACH is formed by the reaction of acetone with a mixed cyanide composition comprising an admixture of a metal cyanide composition and an HCN composition.

The metal cyanide composition preferably comprises an alkali metal cyanide, such as NaCN or KCN, or an alkaline earth metal cyanide, such as Ca(CN)₂ or Mg(CN)₂, most preferably NaCN. The metal cyanide composition may contain a metal hydroxide stabilizer, e.g., NaOH. The metal cyanide composition may also contain water. Suitable forms of the metal cyanide composition, for use in the present invention, include:
aqueous solution - e.g., commercially available as 30% aqueous NaCN solution (Cyanco, Winnemucca, NV);
slurry (e.g., U.S. Patent No. 4,902,301);
paste (e.g., U.S. Patent No. 6,183,710);
wet cake (e.g., U.S. Patent No. 6,162,263);
briquettes and granules (e.g., U.S. Patent Nos. 5,914,075, 5,674,617, and 5,958,588)-e.g., commercially available as solids at 95 to 99 wt% NaCN (DuPont (Wilmington, DE) and Degussa (Mobile, AL)).

It is preferred to transport dry or non-aqueous metal cyanide and to rehydrate the cyanide when ready to use (this providing improved "shelf-life" and being safer to ship than aqueous cyanide). It should be borne in mind that commercial dry metal cyanide is shipped to consumers in specially designed containers which exclude exposure to atmospheric air. (Anhydrous NaCN is hygroscopic, and it can absorb substantial quantities of water causing serious shipping and storage problems due to caking.) One embodiment would be to receive dry metal cyanide in railcars; when the metal cyanide was needed, it would be "rehydrated" to nominal 30 wt% to 40 wt% solution by adding a liquid comprising water directly to the railcar and then offloading the resulting solution. Examples of a suitable liquid comprising water include deionized water, recycle from the ACH purification process (i.e., a water/acetone/HCN stream - prior to its addition to the ACH reactor) or a combination of these.

The metal cyanide is preferably low in carbonate and formate content ( less than 0.5 wt% each, as measured in dry solid metal cyanide); preferably has a minimal nitrile content and is low in other metals, especially iron.

The metal cyanide is preferably low in chloride content (especially if the metal cyanide is NaCN, commercially available "low chloride" NaOH can be used to produce NaCN or the NaCN may be made directly from Trona as taught in European Published Patent Application No. 0 360 555 A1) - this minimizes the potential for chloride-induced stress corrosion cracking in stainless steel ACH production process equipment.

The HCN composition may contain water, although HCN of at least 90 wt% purity is preferred, with at least 95 wt% purity being especially preferred. The HCN composition may also contain one or more acid stabilizers, e.g., organic acids or inorganic acids, such as acetic acid or H₂SO₄ or H₃PO_{4.}

The metal cyanide composition may comprise from 0.5 to 99.5 wt% of the total of the metal cyanide composition and the HCN composition.

Optionally, water may also be fed to the reactor. Such water may take any useable form, e.g., process water, fresh deionized water, water present in material recycled from the ACH purification system( this recycled material may also contain acetone and HCN) or water present in the metal cyanide composition.

Fresh acetone and the mixed cyanide composition are fed continuously to the reactor, which is cooled (cooling may be effected by any means, e.g., by internal coils in the reactor, by a jacket about the reactor or by circulation of the reaction mixture through a heat exchanger, preferably, by circulation of the reaction mixture through an external heat exchanger which also aids mixing; and, typically, the temperature will be maintained between 0°C and 50°C, preferably between 10°C and 40°C) and mixed (preferably with a reactor residence time of from 15 to 120 minutes, e.g., from 20 to 80 minutes) to maximize efficiency.

The pH of the reaction is monitored and maintained at greater than or equal to 7.0, preferably between 7.0 and 8.0, most preferably between 7.3 and 7.8. At a pH below 7.0, the reaction may stall and attempts to increase the pH may result in a runaway reaction. At a pH above 8.0, the ACH may decompose.

Various methods are known in the art for monitoring the pH of the reaction mixture, and any such method may be utilized, including on-line pH probes, direct sampling and titration and adiabatic temperature calculations. It should be borne in mind that the pH of the reaction mixture will be affected by the acid content of the HCN composition and the base content of the metal cyanide composition. As would be obvious to one of ordinary skill in the art, the pH may be lowered by the addition of an acid, e.g., an organic acid or an inorganic acid, such as acetic acid or H₂SO₄ or H₃PO₄. Conversely, the pH may be raised by the addition of a base, such as a metal hydroxide, e.g., NaOH, KOH, Ca(OH)₂ or Mg(OH)₂; a basic ion exchange resin; an amine such as an alkyl amine, e.g., trimethylamine, triethylamine or tributylamine, or an alkanol amine, e.g., triethanolamine; a basic salt, e.g., K₂CO₃; or a basic buffer such as acetic acid-sodium acetate.

Acetone is added to the reactor in at least stoichiometric amount, preferably in excess to improve the yield.

Because the reaction is reversible under basic conditions, the crude ACH product must be stabilized with acid, e.g., an organic acid or an inorganic acid, preferably sulfuric acid. Sufficient acid should be added to acidify the crude ACH, i.e. to lower the pH below 7.0, preferably to the range of between 1.0 and 2.5.

Depending on the type of material used to maintain the basic pH of the reaction system, salts may form as a result of the acid addition that require removal from the stabilized crude ACH. This removal may typically be accomplished by filtration.

Purification of the stabilized crude ACH is then performed, typically, by one or more distillations, to yield a product ACH of greater than 90% purity, preferably greater than 98% purity, which may be used in the production of methacrylates. Such purification may be effected by feeding the stabilized crude ACH to a light-ends stripping column where HCN, acetone and some water are removed overhead and recycled to the reactor. The concentrated ACH bottoms are the sent to a dehydration column where the remaining water is removed under vacuum. Additional removal of concentrated salts may also be effected by filtration during the purification.

In an alternative approach, U.S. Patent No. 3,700,718 discloses an ACH process using filtration; wherein, once filtered, the crude ACH is then purified by stripping unreacted acetone and hydrogen cyanide from the crude ACH with an inert gas to recover product ACH; the resultant inert gas containing unreacted materials is then returned to the ACH reactor, contacted with the reaction mixture, and then recycled to the stripping step.

In a further alternative, U.S. Patent No. 4,130,580 discloses a process for making ACH wherein sodium from NaOH is removed from the crude ACH through the use of an ion exchange resin, prior to stabilization with sulfuric acid. Purification of the ACH is then performed in successive vacuum distillation steps, which serve to remove water, unreacted acetone and HCN from the crude ACH. These components are recycled to the ACH reactor to improve the overall ACH process yield.

### Example

5,440 lbs/hr of a 30% aqueous NaCN solution (1,623 lbs/hr of NaCN, 3,787 lbs/hr of water and 30 lbs/hr of excess caustic (NaOH)), 10,000 lbs/hr of an HCN feed (9,900 lbs/hr of HCN, 85 lbs/hr of water and 15 lbs/hr of H₂SO₄), 20,710 lbs/hr of a fresh acetone feed (20, 710 lbs/hr of acetone), 9,355 lbs/hr of a recycle acetone feed ( 5,225 lbs/hr of acetone, 2,736 lbs/hr of water and 1394 lb/hr of HCN) and 5,465 lbs/hr of a 30% sulfuric acid stream (1,639.5 lbs/hr of H₂SO₄ and 3,825.5 lbs/hr of water) are fed to a reactor. The resultant pH of the reaction mixture is 7.3. The reactor is maintained at a temperature of 10°C by circulation of the reaction mixture through an external heat exchanger. The residence time within the reactor is 90 minutes. A crude acetone cyanohydrin product stream of 50,970 lbs/hr is withdrawn from the reactor and mixed with sulfuric acid to stabilize the same at a pH of 1.8. The stabilized product stream is subjected to filtration, to recover insoluble sodium salts, and distillation to recover acetone cyanohydrin. The overall yield of product ACH is greater than 95% on both an HCN and an acetone basis.

## Claims

1. A process for the production of acetone cyanohydrin, the process comprising:
(A) feeding a metal cyanide composition to a reactor;
(B) feeding an HCN composition to said reactor;
(C) feeding acetone to said reactor;
(D) maintaining a reactor temperature of between 0°C and 50°C in said reactor;
(E) maintaining a pH of at least 7.0 in said reactor;
(F) maintaining a residence time in said reactor of between 15 and 120 minutes;
(G) recovering a product stream from said reactor comprising acetone cyanohydrin.

2. The process according to claim 1, wherein said product stream is acidified.

3. The process according to claim 2, wherein said product stream is acidified to a pH of between 1 and 2.5.

4. The process according to claim 2, wherein said acidified product stream is purified to produce acetone cyanohydrin of greater than 90% purity.

5. The process according to claim 1, wherein said reactor temperature is maintained between 10°c and 40°C.

6. The process according to claim 1, wherein said reactor pH is maintained between 7.0 and 8.0.

7. The process according to claim 6, wherein said reactor pH is maintained between 7.3 and 7.8.

8. The process according to claim 1, wherein said metal cyanide composition comprises from 0.5 to 99.5 wt% of the total of said metal cyanide composition and said HCN composition.

9. In a process for the production of acetone cyanohydrin by the reaction of acetone and HCN under basic pH conditions, the improvement comprising: supplying a metal cyanide composition and an HCN composition to the reaction.

10. The process according to claim 9, wherein said metal cyanide compositon comprises from 0.5 to 99.5 wt% of the total of said metal cyanide composition and said HCN composition.
